(19) Europäisches Patentamt
European Patent Office
Office européen des brevets

(11) EP 3 621 576 B1

(12) FASCICULE DE BREVET EUROPEEN

(45) Date de publication et mention de la délivrance du brevet:
**29.12.2021 Bulletin 2021/52**

(21) Numéro de dépôt: **18724525.3**

(22) Date de dépôt: **11.05.2018**

(51) Int Cl.:
***A61K 8/25*** (2006.01) ***A61K 8/26*** (2006.01)
***A61K 8/29*** (2006.01) ***A61Q 17/04*** (2006.01)
***A61K 8/02*** (2006.01)

(86) Numéro de dépôt international:
**PCT/EP2018/062204**

(87) Numéro de publication internationale:
**WO 2018/206764 (15.11.2018 Gazette 2018/46)**

(54) **COMPOSITION PHOTOSTABLE A BASE DE PARTICULES COMPOSITES DE PERLITE/TITANE/SILICE**

PHOTOSTABILE ZUSAMMENSETZUNG ENTHALTEND KOMPOSITTEILCHEN AUS PERLITE/TITANIUM/SILICA

PHOTOSTABLE COMPOSTION COMPRISING COMPSITE PARTICLES OF PERLITE/TITANIUM/SILICA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.05.2017 FR 1754213**

(43) Date de publication de la demande:
**18.03.2020 Bulletin 2020/12**

(73) Titulaire: **L'OREAL**
**75008 Paris (FR)**

(72) Inventeurs:
- **CANDAU, Didier**
  **94152 Chevilly Larue (FR)**
- **MAGNAN, Anne**
  **94152 Chevilly Larue (FR)**
- **SAFOUANE, Mahassine**
  **94152 Chevilly Larue (FR)**

(74) Mandataire: **Lavoix**
**2, place d'Estienne d'Orves**
**75441 Paris Cedex 09 (FR)**

(56) Documents cités:
**WO-A1-2014/191324** **WO-A1-2015/083482**
**FR-A1- 2 982 148** **KR-A- 20070 002 291**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne une composition comprenant, notamment dans un milieu cosmétiquement acceptable:

a) au moins une phase aqueuse ; et
b) des particules composites de taille moyenne élémentaire en nombre supérieure à 0,1 μm comprenant :

i) une matrice en perlite,
ii) au moins un filtre UV inorganique, et
iii) un enrobage de silice, ladite silice représentant au moins 1% en poids par rapport au poids total de la particule composite.

Il est connu que les radiations lumineuses de longueur d'ondes comprises entre 280 nm et 400 nm permettent le brunissement de l'épiderme humain. Cependant, les rayons de longueur d'ondes plus particulièrement comprises entre 280 et 320 nm, connues sous la dénomination UV-B, provoquent des érythèmes et des brûlures cutanées qui peuvent nuire au développement du bronzage naturel.

Pour ces raisons, ainsi que pour des raisons esthétiques, il existe une demande constante de moyens de contrôle de ce bronzage naturel en vue de contrôler la couleur de la peau : il convient donc de filtrer le rayonnement UV-B.

Il est également connu que les rayons UV-A, de longueur d'ondes comprise entre 320 et 400 nm, et qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré.

Ainsi, il est souhaitable de filtrer aussi le rayonnement UV-A.

De nombreuses compositions photoprotectrices ont été proposées à ce jour pour protéger des effets induits par les UVA et/ou UVB. Ces compositions contiennent généralement des filtres organiques ou minéraux, plus particulièrement des mélanges de filtres liposolubles organiques et/ou des filtres solubles dans l'eau, combinés avec des pigments d'oxydes métalliques tels que le dioxyde de titane ou l'oxyde de zinc. Ces particules inorganiques permettent d'augmenter la protection solaire, ce qui réduit la quantité de filtres organiques et améliore ainsi la cosméticité des formules.

Si les filtres minéraux comme le dioxyde de titane ou l'oxyde de zinc sont largement utilisés en cosmétique pour leurs propriétés absorbantes des UV, ils provoquent cependant un blanchiment lors de leur application sur la peau, ce qui n'est pas esthétique.

En outre, l'absorption dans l'UV engendre des phénomènes indésirables d'oxydoréduction de ces filtres minéraux, qui se manifestent par un changement de couleur de la composition. On note en effet un bleuissement ou un jaunissement et une oxydation des autres composants. C'est ce que l'on appelle l'activité photocatalytique. Cette activité photocatalytique peut induire des réactions non désirables pour la peau et pour les composants présents dans la formulation.

Afin de diminuer l'activité photocatalytique tout en gardant une absorption efficace des UV, on peut utiliser un enrobage organique ou minéral pour ces filtres minéraux. Parmi les enrobages couramment utilisés, on trouve principalement la silice ou l'alumine, mais aussi des substances organiques telles que la cyclométhicone, la diméthicone ou l'acide stéarique. Cela est par exemple décrit dans le brevet US5562990, qui divulgue des particules de dioxyde de titane fluorées comprenant une couche d'alumine, lesdites particules étant traitées avec un composé organosiliconé. Ces traitements ont un profil environnemental non souhaitable, en particulier les traitements avec cyclométhicone, diméthicone ou les organosilicones.

Il existe un besoin constant pour des compositions photoprotectrices efficaces qui soient photo-stables, et qui présentent un blanchiment diminué à l'application.

Les inventeurs ont découvert qu'en utilisant des particules composites à base de TiO2 et de perlite et protégées par un traitement de surface constitué de silice, il était possible d'obtenir des compositions photoprotectrices particulièrement stables aux rayonnements UV et ne provoquant pas l'oxydation des autres composants présents dans la composition. De telles compositions présentent une protection solaire efficace, et sont moins blanches (blanchiment diminué) à l'application.

La présente invention concerne ainsi une composition comprenant, notamment dans un milieu cosmétiquement acceptable :

a) au moins une phase aqueuse ; et
b) des particules composites de taille moyenne élémentaire en nombre supérieure à 0,1 μm comprenant :

i) une matrice en perlite,
ii) au moins un filtre UV inorganique, et
iii) un enrobage de silice, ladite silice représentant au moins 1% en poids par rapport au poids total de la particule composite.

Elle concerne également un procédé cosmétique de soin et/ou de maquillage des matières kératiniques humaines, notamment la peau du corps ou du visage ou des cheveux, comprenant au moins l'application sur lesdites matières kératiniques d'une composition telle que définie précédemment.

Par « matières kératiniques humaines », on entend la peau (corps, visage, contour des yeux), cheveux, cils, sourcils, poils, ongles, lèvres et/ou muqueuses.

Par « milieu cosmétiquement acceptable », on entend tout milieu compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

On entend par « filtre UV inorganique » une molécule ne comprenant pas dans sa structure d'atome de carbone et capable de filtrer le rayonnement UV entre 280 et 400nm.

Par « taille moyenne », on entend la taille moyenne en nombre ou le diamètre moyen en nombre des particules. Les tailles moyennes des particules peuvent être mesurées par diffusion statique de la lumière au moyen d'un granulomètre laser par exemple MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.

Par « taille moyenne élémentaire », on entend la taille de particules non agrégées.

Particules composites

Les particules composites conformes à l'invention comprennent une matrice constituée de particules de perlite et au moins un filtre UV inorganique.

Les particules composites utilisables selon l'invention peuvent être monocouche ou multicouches.

Les particules composites utilisables selon l'invention peuvent être sphériques ou non sphériques, de préférence non sphériques.

Par « non sphériques », on entend des particules en trois dimensions (longueur, largeur, épaisseur ou hauteur) pour lesquelles le rapport de la dimension la plus grande sur la plus petite dimension est supérieur à 1,2. Elles comprennent les particules de forme parallélépipédique (surface rectangulaire ou carrée), discoïdale (surface circulaire) ou ellipsoïdale (surface ovale), caractérisées par trois dimensions : une longueur, une largeur et une hauteur. Quand la forme est circulaire, la longueur et la largeur sont identiques et correspondent au diamètre d'un disque, tandis que la hauteur correspond à l'épaisseur du disque. Quand la surface est ovale, la longueur et la largeur correspondent respectivement au grand axe et au petit axe d'une ellipse et la hauteur correspond à l'épaisseur du disque elliptique formé par la plaquette. Quand il s'agit d'un parallélépipède, la longueur et la largeur peuvent être de dimensions identiques ou différentes : quand elles sont de même dimension, la forme de la surface du parallélépipède est carrée ; dans le cas contraire, la forme est rectangulaire. Quant à la hauteur, elle correspond à l'épaisseur du parallélépipède.

Selon une première variante, les particules composites contiennent une matrice constituée de particules de perlite et dans laquelle sont incluses des particules de filtre UV inorganique. Selon cette réalisation, la matrice présente des inclusions et des particules de filtre UV inorganique sont placées dans les inclusions de la matrice.

De manière préférentielle, les particules composites de l'invention présentent une taille moyenne élémentaire en nombre variant entre 0,1 et 30 $\mu$m, de préférence entre 1 et 28 $\mu$m, mieux entre 3 et 25 $\mu$m.

De manière préférentielle, lorsque les particules composites de l'invention sont sphériques, leur taille moyenne élémentaire en nombre varie entre 0,1 et 30 $\mu$m.

De manière préférentielle, lorsque les particules composites de l'invention sont non sphériques, elles se caractérisent par une taille moyenne élémentaire en nombre de 0,1 à 30 $\mu$m.

Les particules composites non-sphériques utilisables selon l'invention seront de préférence plaquettaires. Par « plaquettaire », on entend une forme parallélépipédique.

Les particules composites plaquettaires présentent de préférence une taille moyenne élémentaire en nombre de 0,1 à 30 $\mu$m.

Les particules composites selon l'invention sont présentes, de préférence, dans les compositions de l'invention dans des concentrations allant de 1 à 70%, de préférence 1,5 à 50%, de manière préférée de 2 à 40% en poids par rapport au poids total de la composition.

*Filtres UV inorganiques*

Les particules composites selon l'invention comprennent au moins un filtre UV inorganique.

Les filtres UV inorganiques sont préférentiellement des pigments d'oxyde métallique. Plus préférentiellement, les filtres UV inorganiques de l'invention sont des particules d'oxyde métallique ayant une taille moyenne de particule élémentaire inférieure à 0,1 μm.

Le filtre UV inorganique est généralement choisi parmi les oxydes métalliques, de préférence les oxydes de titane, de zinc, de fer ou leurs mélanges, et plus particulièrement parmi le dioxyde de titane, l'oxyde de zinc et leurs mélanges. De manière particulièrement préférée, le filtre UV inorganique est le dioxyde de titane (TiO2).

En particulier, le dioxyde de titane (TiO2) peut se présenter sous forme rutile et/ou anatase et/ou sous une forme amorphe.

La teneur massique en filtre UV inorganique dans les particules composites de l'invention est de préférence de 1% à 70% en poids, mieux de 1,2% à 65% en poids, et encore mieux de 1,5% à 60% en poids par rapport au poids total d'une particule composite.

*Particules de perlite*

Les particules composites selon l'invention comprennent une matrice constituée de particules de perlite.

La perlite est un verre naturel d'origine volcanique, de couleur gris-clair ou noir brillant, résultant du refroidissement rapide de la lave et qui se présente sous forme de petites particules ressemblant à de la perle.

Les perlites utilisables selon l'invention sont généralement des aluminosilicates d'origine volcanique et ont comme composition :

70,0-75,0% en poids de silice SiO2
12,0-15,0% en poids d'oxyde d'aluminium Al2O3
3,0-5,0% d'oxyde de sodium Na2O
3,0-5,0% d'oxyde de potassium K2O
0,5-2% d'oxyde de fer Fe2O3
0,2-0,7% d'oxyde de magnésium MgO
0,5-1,5% d'oxyde de calcium CaO, et
0,05- 0,15% d'oxyde de titane TiO2.

De manière préférentielle, les particules de perlite utilisées selon l'invention seront sous forme expansée poreuse.

La perlite est broyée, séchée puis calibrée dans une première étape. Le produit obtenu dit Perlite Ore est de couleur grise et de taille de l'ordre de 100 μm. La Perlite Ore est ensuite expansée (1000°C/2 secondes) pour donner des particules plus ou moins blanches. Lorsque la température atteint 850-900°C, l'eau emprisonnée dans la structure du matériau se vaporise et entraîne l'expansion du matériau par rapport à son volume d'origine. Les particules de perlite expansées conformes à l'invention peuvent être obtenues par le procédé d'expansion décrit dans le brevet US 5,002,698.

De préférence, les particules de perlite utilisées seront broyées ; elles sont dans ce cas dites Expanded Milled Perlite (EMP).

Elles ont de préférence une taille de particule définie par un diamètre médian D50 allant de 0,5 à 50 μm et de préférence de 0,5 à 40 μm.

De préférence, les particules de perlite selon l'invention ont une distribution de tailles de particules telle qu'au moins 50% des particules ont une taille inférieure à 20 μm. En outre, elles ont préférentiellement une distribution de tailles de particules telle que 90% en poids des particules ont une taille inférieure à 55 μm et de préférence une taille inférieure à 40 μm. On préfère par ailleurs que 90% en poids des particules aient une taille supérieure à 5 μm.

De préférence, les particules de perlite utilisées présentent une densité apparente non tassée à 25°C allant de 10 et 400 kg/m3 (Norme DIN 53468) et de préférence de 10 et 300 kg/m3.

On utilisera en particulier les particules de perlite expansée vendues sous les noms commerciaux OPTIMAT 1430 OR® ou OPTIMAT 2550® par la société WORLD MINERALS ou les produits commerciaux GK-110 THIN® et GK-110 EXTRA THIN® par la société LANGFANG XINDAZHONG FILTER et la société HENAN ZHONGNAN FILTER AID.

La teneur massique en perlite dans les particules composites de l'invention est de préférence de 10 à 99% en poids, plus préférentiellement de 20 à 98% en poids et encore plus préférentiellement 30 à 97% en poids par rapport au poids total d'une particule composite.

*Préparation des particules composites*

Les particules composites conformes à la présente invention peuvent être obtenues selon le procédé décrit dans le brevet FR2882371B1 consistant à imprégner des particules de perlite par une suspension aqueuse de particules d'oxyde métallique tels que le dioxyde de titane ayant une taille moyenne de particule élémentaire en nombre inférieure à 0,1 $\mu$m, puis à réduire les précurseurs au sein dudit matériau formant la matrice ; l'imprégnation étant effectuée sous pression de vapeur saturante et sous reflux de la solution d'un ou plusieurs précurseurs d'oxyde métallique (par exemple le dioxyde de titane) et la réduction étant effectuée par voie radiolytique.

Les précurseurs d'oxyde métallique peuvent être choisies parmi des sels minéraux (par exemple des sulfates, des perchlorates), des sels organiques (par exemple formiates, néodécanoates) ou des composés organométalliques.

La solution de précurseur(s) peut contenir en outre un agent intercepteur de radicaux oxydants, qui intercepte les radicaux oxydants formés dans la solution lors de l'irradiation, ce qui évite l'oxydation des particules colloïdales produites. L'intercepteur de radicaux oxydants est choisi de préférence parmi les alcools primaires, les alcools secondaires et les formiates.

Les particules composites à base de perlite et de filtre inorganique peuvent être également obtenues selon le procédé décrit dans la demande WO 2006/083326 qui consiste à générer une dispersion gazeuse en écoulement comprenant des gouttelettes d'un support précurseur dispersé en phase gazeuse. Le support précurseur contient un véhicule liquide et au moins un précurseur d'oxyde métallique de taille élémentaire inférieure à 0,1 $\mu$m et une matrice à base de perlite. Les particules composites sont formées à partir de la dispersion gazeuse en éliminant une portion du véhicule liquide des gouttelettes du milieu précurseur.

Selon une forme particulièrement préférée de l'invention, les particules composites peuvent être préparées également selon le procédé décrit dans la demande KR1020000069638 qui consiste, selon une méthode sol-gel, à mélanger dans de l'eau une suspension aqueuse d'alcoxyde métallique (notamment d'alcoxyde de titane tel que le tétra-n-butyl oxyde de titane encore appelé tétra-n-butyl-titanate (TNBT)) à une matrice de perlite et à faire réagir par hydrolyse l'alcoxyde métallique pendant une durée allant de préférence de 1 à 8 heures à une température allant de préférence de 30 à 78°C. Ensuite, la suspension ainsi obtenue est filtrée, lavée et séchée pour produire une poudre composite de perlite revêtue d'oxyde de métallique. Pour améliorer l'uniformité du revêtement de filtre inorganique sur les particules de perlite, on peut faire réagir selon le même procédé les particules composites obtenues avec la suspension d'alcoxyde métallique.

Selon une autre forme particulière de l'invention, les particules composites peuvent être préparées également selon le procédé décrit dans le brevet U56447759, qui consiste à préparer une suspension de particules de perlite, en ajoutant un agent de complexation dans la suspension, en ajoutant un sel d'oxyde métallique précurseur du filtre inorganique et un agent alcalin du type carbonate pour former des particules de carbonate d'oxyde métallique basiques sur la matrice de perlite et en calcinant les particules composites.

Selon une forme particulière de l'invention, les particules composites peuvent être préparées également selon le procédé décrit dans la demande JP2008115161, consistant à préparer une suspension de particules de perlite à pH acide en présence d'acide chlorhydrique. On ajoute ensuite les particules d'oxyde métallique constituant le filtre inorganique. A pH acide, un agent floculant comme le chlorure de calcium est ajouté pour obtenir les particules composites.

Selon une autre forme particulière de l'invention, les particules composites de l'invention peuvent être préparées en soumettant les particules de perlite et le ou les filtre inorganiques tels que définis précédemment à un procédé de fusion méchanochimique.

Un procédé de fusion méchanochimique signifie un procédé dans lequel on exerce sur une pluralité de composés une force mécanique telle qu'un choc, une force de friction, une force de cisaillement, pour produire une fusion partielle des différents composés.

Le procédé de fusion méchanochimique peut être mis en œuvre, par exemple, avec un appareil comprenant une chambre rotative et une pièce fixée interne avec un grattoir, tel que le dispositif de mécanofusion de la marque commerciale 20 Hosokawa Micron Corporation® au Japon.

Il est préférable d'utiliser un procédé dit hybrideur (hybridizer) comme procédé de fusion mécanochimique.

Le procédé hybrideur a été développé dans les années 1980. Le procédé hybrideur est une catégorie de procédés de fusion mécanochimique dans lequel une force mécanique élevée est appliquée sur une pluralité de particules pour produire une réaction mécanochimique et former une particule composite.

Selon le procédé hybrideur, la force mécanique est exercée par un rotor à haute vitesse pouvant avoir un diamètre de 10 cm à 1 m, et peut tourner à une vitesse de 1000 tours/minute à 100000 tours/minute. Ainsi, le procédé hybrideur peut être défini comme un procédé de fusion mécanochimique utilisant un rotor à haute vitesse. Le procédé hybrideur est effectué en présence d'air ou dans des conditions sèches. En effet, en raison de la vitesse élevée du rotor, un flux d'air de haute vitesse peut être produit près du rotor. Certains matériaux liquides peuvent être soumis au procédé hybrideur avec des matériaux solides. Le terme « procédé hybrideur » a été utilisé comme terme technique dans la présente description.

Le procédé hybrideur peut être effectué en utilisant un système d'hybridation de marque Nara Machinery® au Japon, dans lequel les particules de perlite et celles de filtre inorganique sont introduites dans un hybrideur équipé d'un rotor à haute vitesse muni d'une pluralité de lames dans une chambre sous conditions sèches. Les particules sont dispersées dans la chambre et une énergie mécanique et thermique (i.e. compression, friction, contrainte de cisaillement) sont exercées sur les particules pendant une courte période de temps telle que de 1 à 10 minutes, de préférence de 1 à 5 minutes. On obtient un type de particules (i.e. de fines particules) intégrées ou fixées sur un autre type de particules (particules noyaux) pour former les particules composites conformes à l'invention. Il est préférable que les particules aient été soumises à un ou plusieurs traitements électrostatiques comme un tremblement pour former un « mélange ordonné » dans lequel un type de particules est étalé pour recouvrir l'autre type de particules.

Le procédé hybrideur peut être aussi mis en œuvre en utilisant un composteur thêta de marque Tokuju Corporation@ au Japon. Le procédé hybrideur peut être effectué avec un dispositif Composi Hybrid ou Mechano Hybrid vendus par la société Nippon Coke.

Selon la présente invention, les particules de perlite, le ou les filtres inorganiques peuvent être introduits dans un hybrideur pour former un pigment composite. Le procédé hybrideur peut être mis en œuvre en utilisant un rotor tournant à 8000 tours/minute (100 m/sec) pendant environ 3 minutes.

De plus, le procédé hybrideur peut créer un ensemble ordonné (revêtement uniforme) du ou des filtres UV inorganiques sur la particule de perlite et produire de fortes liaisons sur la surface de la particule de perlite et la couche de revêtement comprenant le ou les filtres UV inorganiques.

Il est à noter que le procédé hybrideur est très différent des autres procédés utilisant par exemple moulin à billes et un broyeur à jet. En effet, les moulins à billes provoquent une pulvérisation ou une agglomération des particules formant le cœur du composite et les broyeurs à jet provoquent une pulvérisation desdites particules rendant difficile le revêtement des fines particules de manière uniforme sur les particules formant le cœur du composite.

*Enrobage de silice*

Les particules composites selon l'invention comprennent également un enrobage de silice, qui représente au moins 1% en poids par rapport au poids total de la particule composite. Cette silice constitue l'enrobage des particules composites. Elle est donc distincte de la silice comprise dans la matrice de perlite.

En effet, les particules composites selon l'invention ont typiquement subi un ou plusieurs traitements de surface de nature chimique, électronique, mécanochimique et/ou mécanique avec des composés à base de silice (SiO2).

Ainsi, les particules composites selon l'invention comprennent typiquement une matrice en perlite, au moins un filtre UV inorganique, et ont subi un traitement de surface (ou enrobage) avec de la silice, cette silice représentant au moins 1% en poids par rapport au poids total de la particule composite.

La teneur massique en silice (i.e. l'enrobage) dans les particules composites de l'invention est de préférence d'au moins 2% en poids, de préférence comprise entre 1 et 10% en poids, plus préférentiellement entre 2 et 8% en poids et encore plus préférentiellement entre 2,5 et 6% en poids par rapport au poids total d'une particule composite.

Selon un mode de réalisation, une fois que les particules composites comprenant la matrice de perlite et le filtre UV inorganique sont obtenues, notamment par l'un des procédés décrits ci-dessus, elles subissent un traitement de surface avec de la silice, cette silice représentant au moins 1% en poids par rapport au poids total de la particule composite. Les particules ainsi traitées sont ensuite lavées, séchées et filtrées.

De préférence, les particules composites selon l'invention peuvent être préparées selon l'un des exemples A ou B :

Exemple de préparation A

100 g de poudre de perlite vendus sous la dénomination commerciale GK-110 Thin® par la société Langfang Xindazhong Filter et la société Henan Zhongnan Filter Aid ont été placés dans un ballon équipé d'un agitateur et 150 g de tétra-n-butoxyde de titane (Super Urecoat Industries) sous la forme d'une suspension ont été ajoutés. La perlite a été conçue pour absorber le précurseur de titane. Une quantité de 0,2 à 1 litre d'eau a ensuite été ajoutée pour permettre une réaction de 1 à 8 heures à 30-100°C. Ensuite, la suspension ainsi obtenue a été filtrée, lavée et séchée pour produire une poudre composite de perlite revêtue de dioxyde de titane.

Dans une deuxième étape, on a placé 100 g de cette poudre composite dans un ballon et on a ajouté 150 g de tétra-n-butoxyde de titane sous forme de suspension. Le composite a ensuite été réalisé pour absorber le précurseur de titane. Une quantité de 0,2 à 1 litre d'eau a ensuite été ajoutée pour permettre une réaction de 1 à 8 heures à 30-100°C. Ensuite, la suspension ainsi obtenue a été filtrée, lavée et séchée pour produire une poudre composite de perlite revêtue de dioxyde de titane. Enfin, on a obtenu une poudre composite contenant 40% en poids de dioxyde de titane.

Cette poudre composite a ensuite été enrobée avec au moins 1% de silice en poids par rapport au poids total de la particule composite.

Exemple de préparation B

Une poudre composite à base de perlite et de dioxyde de titane a été préparée, dans les mêmes conditions que dans l'exemple A, en utilisant une poudre de perlite vendue sous la dénomination commerciale GK-110 Extra Thin par la société Langfang Xindazhong Filter et la société Henan Zhongnan Filter Aid.
Cette poudre composite a ensuite été enrobée avec au moins 1% de silice en poids par rapport au poids total de la particule composite.

Phase aqueuse

Les compositions selon l'invention comprennent au moins une phase aqueuse.
La phase aqueuse contient de l'eau, et éventuellement au moins un solvant organique soluble ou miscible dans l'eau.
Une phase aqueuse convenant à l'invention peut comprendre, par exemple, une eau choisie parmi une eau de source naturelle, telle que l'eau de La Roche-Posay, l'eau de Vittel, ou les eaux de Vichy, ou une eau florale.
Les solvants solubles ou miscibles dans l'eau convenant à l'invention comprennent les monoalcools à chaîne courte par exemple en C1-C4 comme l'éthanol, l'isopropanol; les diols ou les polyols comme l'éthylène glycol, le 1,2-propylène glycol, le 1,3-butylène glycol, l'hexylèneglycol, le diéthylèneglycol, le dipropylène glycol, le 2-éthoxyéthanol, le diéthylène glycol monométhyléther, le triéthylène glycol monométhyléther, le glycérol, le sorbitol, et leurs mélanges.
Selon un mode préféré, on pourra utiliser plus particulièrement l'éthanol, le propylène glycol, la glycérine, ou l'un de leurs mélanges.
Selon une forme particulière de l'invention, la phase aqueuse représente de 15 à 95% en poids, préférentiellement de 30 à 80% en poids, encore mieux de 40 à 70% en poids par rapport au poids total de la composition.

Phase huileuse

Les compositions conformes à l'invention comprennent de préférence au moins une phase huileuse.
Au sens de l'invention on entend par « phase huileuse », une phase comprenant au moins une huile et l'ensemble des ingrédients liposolubles et lipophiles et des corps gras utilisés pour la formulation des compositions de l'invention.
On entend par huile, tout corps gras sous forme liquide à température ambiante (20 - 25°C) et à pression atmosphérique (760 mm Hg). Une huile convenant à l'invention peut être volatile ou non volatile.
L'huile peut être choisie parmi des huiles hydrocarbonées, des huiles de silicone, des huiles fluorées et leurs mélanges. Une huile hydrocarbonée convenant à l'invention peut être une huile hydrocarbonée animale, une huile hydrocarbonée végétale, une huile hydrocarbonée minérale, ou une huile hydrocarbonée synthétique. Une huile convenant à l'invention peut avantageusement être choisie parmi des huiles hydrocarbonées minérales, des huiles hydrocarbonées végétales, des huiles hydrocarbonées synthétiques, des huiles de silicone et leurs mélanges.
Au sens de la présente invention, on entend par « huile siliconée », une huile comprenant au moins un atome de silicium, et notamment au moins un groupe Si-O.
On entend par « huile hydrocarbonée », une huile contenant principalement des atomes d'hydrogène et de carbone.
On entend par « huile fluorée », une huile comprenant au moins un atome de fluor.
Une huile hydrocarbonée convenant à l'invention peut en outre éventuellement comprendre des atomes d'oxygène, d'azote, de soufre et/ou de phosphore, par exemple, sous la forme de groupes hydroxyle, amine, amide, ester, éther ou acide, et en particulier sous la forme de groupes hydroxyle, ester, éther ou acide.
La phase huileuse comprend en général, outre le ou les filtres UV lipophiles, au moins une huile hydrocarbonée volatile ou non volatile et/ou une huile siliconée volatile et ou non volatile.
Par "huile volatile", on entend au sens de l'invention une huile susceptible de s'évaporer au contact de la peau ou de la fibre kératinique en moins d'une heure, à température ambiante et pression atmosphérique. Le ou les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).
Par "huile non volatile", on entend une huile restant sur la peau ou la fibre kératinique à température ambiante et pression atmosphérique au moins plusieurs heures et ayant notamment une pression de vapeur inférieure à $10^{-3}$ mm de Hg (0,13 Pa).

*Huiles hydrocarbonées*

Comme huiles hydrocarbonées non volatiles utilisables selon l'invention, on peut notamment citer :

(i) les huiles hydrocarbonées d'origine végétale telles que les triesters de glycérides qui sont en général des triesters d'acides gras et de glycérol dont les acides gras peuvent avoir des longueurs de chaînes variées de C4 à C24, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,

(ii) les éthers de synthèse ayant de 10 à 40 atomes de carbone ;

(iii) les hydrocarbures linéaires ou ramifiés, d'origine minérale ou synthétique tels que la vaseline, les polydécènes, le polyisobutène hydrogéné tel que le parléam, le squalane et leurs mélanges ;

(iv) les esters de synthèse comme les huiles de formule RCOOR' dans laquelle R représente le reste d'un acide gras linéaire ou ramifié comportant de 1 à 40 atomes de carbone et R' représente une chaîne hydrocarbonée notamment ramifiée contenant de 1 à 40 atomes de carbone à condition que R + R' soit > 10, comme par exemple l'huile de Purcellin (octanoate de cétostéaryle), le myristate d'isopropyle, le palmitate d'isopropyle, le benzoate d'alcools en C12-C15 comme le produit vendu sous la dénomination commerciale « Finsolv TN » ou « Witconol TN » par la société WITCO ou « TEGOSOFT TN » par la société EVONIK GOLDSCHMIDT, le benzoate de 2-éthylphenyle comme le produit commercial vendu sous le nom « X-TEND 226 » par la société ISP, le lanolate d'isopropyle, le laurate d'hexyle, l'adipate de diisopropyle, l'isononanoate d'isononyle, l'érucate d'oléyle, le palmitate de 2-éthyl-hexyle, l'isostéarate d'isostéaryle, le sébacate de diisopropyle comme le produit vendu sous la dénomination de « Dub Dis » par la société Stearinerie Dubois, des octanoates, décanoates ou ricinoléates d'alcools ou de polyalcools comme le dioctanoate de propylène glycol ; les esters hydroxyles comme le lactate d'isostéaryle, le malate de di-isostéaryle ; et les esters du pentaérythritol ; les citrates ou tartrates comme les tartrates de di-alkyle linéaire en C12-C13 tels que ceux vendus sous le nom COSMACOL ETI par la Société ENICHEM AUGUSTA INDUSTRIALE ainsi que les tartrates de di-alkyle linéaire en C14-C15 tels que ceux vendus sous le nom COSMACOL ETL par la même société ; les acétates ;

(y) les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;

(vi) les acides gras supérieurs tels que l'acide oléique, l'acide linoléique, l'acide linolénique ;

(vii) les carbonates comme le dicaprylyl carbonate comme le produit vendu sous la dénomination « Cetiol CC » par la société Cognis ;

(viii) les amides grasses comme l'isopropyl N-lauroyl sarcosinate comme le produit vendu sous le nom commercial Eldew SL 205 de chez Ajinomoto et leurs mélanges.

Parmi les huiles hydrocarbonées non volatiles utilisables selon l'invention, on préfèrera plus particulièrement les triesters de glycéride et notamment les triglycérides des acides caprylique/caprique, les esters de synthèse et notamment l'isononanoate d'isononyle, le sébacate de diisopropyle, le benzoate d'alcools en C12-C15, le benzoate de 2-éthylphenyle et les alcools gras notamment l'octyldodécanol.

Comme huiles hydrocarbonées volatiles utilisables selon l'invention, on peut notamment citer les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane les huiles vendues sous les noms commerciaux d'Isopars ou de Permetyls, les esters ramifiés en C8-C16, le néopentanoate d'iso-hexyle, et leurs mélanges.

On peut citer aussi les alcanes décrits dans les demandes de brevets de la société Cognis WO2007/068371 ou WO2008/155059 (mélanges d'alcanes distincts et différant d'au moins un carbone). Ces alcanes sont obtenus à partir d'alcools gras, eux-mêmes obtenus à partir d'huile de coprah ou de palme. On peut citer les mélanges de n-undécane (C11) et de n-tridécane (C13) obtenus aux exemples 1 et 2 de la demande WO2008/155059 de la Société Cognis.

On peut également citer le n-dodécane (C12) et le n-tétradécane (C14) vendus par Sasol respectivement sous les références PARAFOL 12-97 et PARAFOL 14-97, ainsi que leurs mélanges.

D'autres huiles hydrocarbonées volatiles comme les distillats de pétrole, notamment ceux vendus sous la dénomination Shell Soit par la société SNELL, peuvent aussi être utilisées.

Selon un mode de réalisation, le solvant volatil est choisi parmi les huiles volatiles hydrocarbonées ayant de 8 à 16 atomes de carbone et leurs mélanges.

*Huiles siliconées*

Les huiles siliconées non volatiles peuvent être choisies notamment parmi les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates .

Comme huiles volatiles siliconées, on peut citer par exemple les huiles de silicones linéaires ou cycliques volatiles, notamment celles ayant une viscosité de 8 centistokes ($8.10^{-6}$ $m^2/s$), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également citer les huiles linéaires alkyltrisiloxanes volatiles de formule générale (I) :

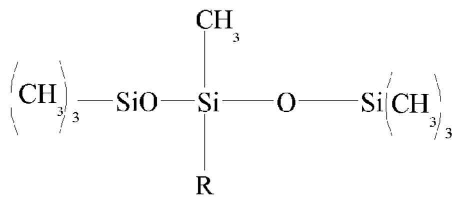

(I)où R représente un groupe alkyle comprenant de 2 à 4 atomes de carbone et dont un ou plusieurs atomes d'hydrogène peuvent être substitués par un atome de fluor ou de chlore.

Parmi les huiles de formule générale (I), on peut citer : le 3-butyl 1,1,1,3,5,5,5-heptaméthyl trisiloxane, le 3-propyl 1,1,1,3,5,5,5-heptaméthyl trisiloxane, et le 3-éthyl 1,1,1,3,5,5,5-heptaméthyl trisiloxane, correspondant aux huiles de formule (I) pour lesquelles R est respectivement un groupe butyle, un groupe propyle ou un groupe éthyle.

*Huiles fluorées*

On peut également utiliser des huiles volatiles fluorées, telles que le nonafluorométhoxybutane le nonafluorométhoxybutane, le décafluoropentane, le tétradécafluorohexane, le dodecafluoropentane et leurs mélanges.

La phase huileuse peut également comprendre d'autres corps gras. Un autre corps gras pouvant être présent dans la phase huileuse peut être, par exemple :

- un acide gras choisi parmi les acides gras comportant de 8 à 30 atomes de carbone, comme l'acide stéarique, l'acide laurique, l'acide palmitique et l'acide oléique ;
- une cire choisi parmi les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ;
- une gomme choisie parmi les gommes de silicone (diméthiconol),
- un composé pâteux, comme les composés silicones polymériques ou non, les esters d'un glycérol oligomère, le propionate d'arachidyle, les triglycérides d'acides gras et leurs dérivés,
- et leurs mélanges.

De façon préférentielle, la phase huileuse représente de 5 à 95% en poids, et préférentiellement de 10 à 80% en poids par rapport au poids total de la composition.

Les compositions selon l'invention peuvent également comprendre en outre au moins un filtre UV organique.

Filtres UV organiques

Les filtres UV organiques sont notamment choisis parmi les composés cinnamiques ; les composés anthranilates ; les composés salicyliques, les composés dibenzoylméthane, les composés benzylidène camphre ; les

composés benzophénone ; les composés diphénylacrylate ; les composés triazine ; les composés benzotriazole ; les composés benzalmalonate notamment ceux cités dans le brevet U55624663; les dérivés de benzimidazole ; les composés imidazolines ; les composés bis-benzoazolyle tels que décrits dans les brevets EP669323 et US 2,463,264; les composés p-aminobenzoïques (PABA) ; les composés méthylène bis-(hydroxyphényl benzotriazole) tels que décrits dans les demandes U55,237,071, US 5,166,355, GB2303549, DE 197 26 184 et EP893119 ; les composés benzoxazole tels que décrits dans les demandes de brevet EP0832642, EP1027883, EP1300137 et DE10162844 ; les polymères filtres et silicones filtres tels que ceux décrits notamment dans la demande WO93/04665; les dimères dérivés d'a-alkylstyrène tels que ceux décrits dans la demande de brevet DE19855649 ; les composés 4,4-diarylbutadiènes tels que décrits dans les demandes EP0967200, DE19746654, DE19755649, EP-A1008586, EP1133980 et EP133981 et leurs mélanges.

Comme exemples d'agents photoprotecteurs organiques, on peut citer ceux désignés ci-dessous sous leur nom INCI :

*Composés cinnamiques:*

Ethylhexyl Methoxycinnamate vendu notamment sous le nom commercial PARSOL MCX par DSM Nutritial Products
Isopropyl Methoxycinnamate
Isoamyl p-Methoxycinnamate vendu sous le nom commercial NEO HELIOPAN E 1000 par Symrise
DEA Methoxycinnamate
Diisopropyl Methylcinnamate
Glyceryl Ethylhexanoate Dimethoxycinnamate.

*Composés para-aminobenzoique :*

PABA,
Ethyl PABA,
Ethyl Dihydroxypropyl PABA,
Ethylhexyl Diméthyl PABA vendu notamment sous le nom « ESCALOL 507®» par ISP,
Glyceryl PABA,
PEG-25 PABA vendu sous le nom « UVINUL P 25®» par BASF.

*Composés salicyliques :*

Homosalate vendu sous le nom « Eusolex HMS » par Rona/EM Industries,
Ethylhexyl Salicylate vendu sous le nom « NEO HELIOPAN OS® » par Symrise,
Dipropyleneglycol Salicylate vendu sous le nom « DIPSAL ®» par SCHER,
TEA Salicylate, vendu sous le nom « NEO HELIOPAN TS® » par Symrise.

*Composés dibenzoylméthane*

Butylmethoxydibenzoylmethane ou Avobenzone vendu notamment sous le nom commercial PARSOL 1789 par DSM Nutritial Products

*Composés $\beta,\beta$-diphénylacrylate :*

Octocrylene vendu notamment sous le nom commercial « UVINUL N 539 » par 40 BASF,
Etocrylene, vendu notamment sous le nom commercial « UVINUL N 35 » par BASF.

*Composés benzophénone :*

Benzophenone-1 vendu sous le nom commercial « UVINUL 400 » par BASF,
Benzophenone-2 vendu sous le nom commercial « UVINUL D 50 » par BASF
Benzophenone-3 ou Oxybenzone, vendu sous le nom commercial « UVINUL M 5 40» par BASF,
Benzophenone-4 vendu sous le nom commercial « UVINUL MS 40 » par BASF,
Benzophenone-5 Benzophenone-6 vendu sous le nom commercial « Helisorb 11®» par Norquay
Benzophenone-8 vendu sous le nom commercial « Spectra-Sorb UV-24® » par 10 American Cyanamid
Benzophenone-9 vendu sous le nom commercial« UVINUL DS 49®» par BASF,
Benzophenone-12 2-(4-diethylamino-2-hydroxybenzoyl)-benzoate de n-hexyle vendu sous le nom commercial « UVINUL A Plus C,» ou en mélange avec l'octylmethoxycinnamate 15 sous le nom commercial « UVINUL A Plus B®» par la société BASF,
1,1'-(1,4-piperazinediy1)bis[14244-(diethylamino)-2-hydroxybenzoyl]pheny1]-methanone (CAS 919803-06-8) tel que décrit dans la demande WO2007/071584; ce composé étant avantageusement utilisé sous forme micronisée (taille moyenne de 0,02 à 2 μm) pouvant être obtenue par exemple selon le procédé de micronisation décrit dans les demandes GB-A-2 303 549 et EP-A-893119 et notamment sous forme de dispersion aqueuse.

*Composés benzylidène camphre :*

3-Benzylidene camphor fabriqué sous le nom « MEXORYL SDC » par CHIMEX,
4-Methylbenzylidene camphor vendu sous le nom « EUSOLEX 6300®» par MERCK,
Benzylidene Camphor Sulfonic Acid fabriqué sous le nom « MEXORYL SLC » par CHIMEX,
Camphor Benzalkonium Methosulfate fabriqué sous le nom « MEXORYL SO® » par CHIMEX,
Terephthalylidene Dicamphor Sulfonic Acid fabriqué sous le nom « MEXORYL SX®» par CHIMEX,
Polyacrylamidomethyl Benzylidene Camphor fabriqué sous le nom « MEXORYL SW® » par CHIMEX.

*Composés phenyl benzimidazole :*

Phenylbenzimidazole Sulfonic Acid vendu notamment sous le nom commercial «EUSOLEX 232®» par MERCK.

*Composés bis-benzoazolyle*

Disodium Phenyl Dibenzimidazole Tetrasulfonate vendu sous le nom commercial «NEO HELIOPAN AP® » par HAARMANN et REIMER.

*Composés phenyl benzotriazole :*

Drometrizole Trisiloxane vendu sous le nom « Silatrizole® » par RHODIA CHIMIE.

*Composés méthylène bis-(hydroxyphényl benzotriazole)*

Methylène bis-Benzotriazolyl Tetramethylbutylphénol notamment sous forme solide comme le produit vendu sous le nom commercial « MIXXIM BB/100 C » par FAIRMOUNT CHEMICAL ou sous forme de dispersion aqueuse de particules micronisées ayant une taille moyenne des particules qui varie de 0,01 à 5pm et plus préférentiellement de 0,01 à 2 μm et plus particulièrement de 0,020 à 2 μm avec au moins un tensioactif alkylpolyglycoside de structure CnH(2n+1)O(C6H10O5)xH dans laquelle n est un entier de 8 à 16 et x est le degré moyen de polymérisation de l'unité (C8H10O5) et varie de 1,4 à 1,6 telle que décrite dans le brevet GB-A-2 303 549 notamment vendue sous le nom commercial « TINOSORB M®» par la société BASF ou sous la forme d'une dispersion aqueuse de particules micronisées ayant une taille moyenne des particules qui varie de 0,02 à 2μm et plus préférentiellement de 0,01 à 1,5 μm et plus particulièrement de 0,02 à 1 μm en présence d'au moins un mono-(C8-C20)alkylester de polyglycérol ayant un degré de polymérisation de glycérol d'au moins telles que les dispersions aqueuses décrites dans la demande WO2009/063392.

*Composés triazine :*

- Bis-Ethylhexyloxyphenol Methoxyphenyl Triazine vendu sous le nom commercial «TINOSORB S®» par BASF,
- Ethylhexyl Triazone vendu notamment sous le nom commercial «UVINUL 25 T150® » par BASF,
- Diethylhexyl Butamido Triazone vendu sous le nom commercial « UVASORB HEB C,» par SIGMA 3V,
- 2,4,6-tris(4'-amino benzalmalonate de dinéopentyle)-s-triazine,
- 2,4,6-tris-(4'-amino benzalmalonate de diisobutyle)-s- triazine,
- 2,4-bis (4'-aminobenzoate de n-butyle)-6-(aminopropyltrisiloxane)-s-triazine,
- 2,4-bis (4'-aminobenzalmalonate de dinéopentyle)-6-(4'-aminobenzoate de nbutyle)-s-triazine,
- les filtres triazines symétriques substituées par des groupes naphthalényles ou des groupes polyphényles décrits dans le brevet U56,225,467, la demande WO2004/085412 (voir composés 6 et 9) ou le document « Symetrical Triazine Derivatives» IP.COM IPC0M000031257 Journal , INC WEST HENRIETTA, NY, US (20 septembre 2004) notamment la 2,4,6-tris(di-phenyl)-triazine et la 2,4,6- tris(ter-phenyl)-triazine qui est repris dans les demandes de brevet WO06/035000, WO06/034982, WO06/034991, WO06/035007, WO2006/034992, 40 WO2006/034985, ces composés étant utilisés avantageusement sous forme micronisée (taille moyenne de particule de 0,02 à 3 µm) pouvant être obtenue par exemple selon le procédé de micronisation décrit dans les demandes GB-A-2 303 549 et EP-A-893119 et notamment en dispersion aqueuse ;
- les silicones triazines substituées par deux groupes aminobenzoates telles que décrites que le brevet EP0841341 en particulier le 2,4-bis-(n-butyl 4'-aminobenzalmalonate)-6-[(3-{1, 3, 3, 3-tetramethy1-1-[(trimethylsilyloxy]-disiloxanyllpropyl)amino]-s-triazine.

*Composés anthraniliques :*

Menthyl anthranilate vendu sous le nom commercial commercial « NEO HELIOPAN MA® » par Symrise.

*Composés imidazolines :*

Ethylhexyl Dimethoxybenzylidene Dioxoimidazoline Propionate,

*Composés benzalmalonate :*

Polyorganosiloxane à fonctions benzalmalonate comme le Polysilicone-15 vendu sous la dénomination commerciale « PARSOL SLX » par HOFFMANN LA ROCHE.

*Composés 4,4-diarylbutadiène :*

-1,1-dicarboxy (2,2'-diméthyl-propyl)-4,4-diphénylbutadiène.

*Composés benzoxazole :*

2,4-bis-[5-1(dim éthylpropyl)benzoxazol-2-y1-(4-pheny1)-imino]-6-(2-ethylhexyl)-imino-1, 3, 5-triazine vendu sous le nom d'Uvasorb K2A® par Sigma 3V.

Les filtres organiques préférentiels sont choisis parmi :

Ethylhexyl Methoxycinnamate
Butylmethoxydibenzoylmethane
Ethylhexyl Salicylate,
Homosalate,
Octocrylene,
Phenylbenzimidazole Sulfonic Acid,
Benzophenone-3,
Benzophenone-4,
Benzophenone-5,
2-(4-diethylamino-2-hydroxybenzoy1)-benzoate de n-hexyle
4-Methylbenzylidene camphor,
Terephthalylidene Dicamphor Sulfonic Acid,

Disodium Phenyl Dibenzimidazole Tetra-sulfonate,
Methylène bis-Benzotriazolyl Tetramethylbutylphénol
Bis-Ethylhexyloxyphenol Methoxyphenyl Triazine
Ethylhexyl Triazone,
Diethylhexyl Butamido Triazone,
2,4,6-tris(4'-amino benzalmalonate de dinéopentyle)-s-triazine
2,4,6-tris-(4'-amino benzalmalonate de diisobutyle)-s-triazine
2,4-bis-(4'-aminobenzoate de n-butyle)-6-(aminopropyltrisiloxane)-s-triazine,
2,4-bis-(4'-aminobenzalmalonate de dinéopentyle)-6-(4'-aminobenzoate de nbutyle)-s-triazine,
2,4-bis-(n-butyl 4'-aminobenzalmalonate)-6-[(3-{1,3,3,3-tetramethyl-1-[(trimethylsilyloxy] disiloxanyllpropyl)amino]-s-triazine,
2,4,6-tris-(di-phenyl)-triazine, 2,4,6-tris-(ter-phenyl)-triazine,
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-y1-(4-pheny1)-imino]-6- (2-ethylhexyl)-imino-1,3,5-triazine
et leurs mélanges.

Les filtres organiques particulièrement préférés sont choisis parmi

Butylmethoxydibenzoylmethane
Ethylhexyl Salicylate,
Homosalate,
Octocrylene,
2-(4-diethylamino-2-hydroxybenzoy1)-benzoate de n-hexyle
Terephthalylidene Dicamphor Sulfonic Acid,
Bis-Ethylhexyloxyphenol Methoxyphenyl Triazine
Ethylhexyl Triazone,
Diethylhexyl Butamido Triazone,
2,4-bis-(n-butyl 4'-aminobenzalmalonate)-6-[(3-{1,3,3,3-tetramethyl-1-[(trimethyl-silyloxy]disiloxanyllpropyl)amino]-s-triazine,
Drometrizole Trisiloxane
et leurs mélanges.

Les filtres organiques, lorsqu'ils sont présents, sont présents à des teneurs allant de 0,01 à 30% en poids et de préférence de 0,1 à 20% en poids par rapport au poids total de la composition de l'invention.

Les compositions conformes à la présente invention peuvent comprendre en outre des adjuvants cosmétiques classiques notamment choisis parmi les épaississants ioniques ou non ioniques, les humectants, les agents anti-mousse, les parfums, les conservateurs, les tensioactifs, des actifs, les charges, les propulseurs, les agents alcalinisants ou acidifiants ou tout autre ingrédient habituellement utilisé dans le domaine cosmétique et/ou dermatologique.

Comme épaississants, on peut citer les polymères carboxyvinyliques tels que les Carbopols® (Carbomers) et les Pemulen comme les Pemulen TR1® et Pemulen TR2® (acrylate/C10-C30 alkylacrylate crosspolymer) ; les polyacrylamides comme par exemple les copolymères réticulés vendus sous les noms Sepigel 305® (nom C.T.F.A. : polyacrylamide/C13-14 isoparaffin/Laureth 7) ou Simulgel 600 (nom C.T.F.A. : acrylam ide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS® » (nom CTFA : ammonium polyacryloyldimethyl taurate) ou le SIMULGEL 800® commercialisé par la société SEPPIC (nom CTFA : sodium polyacryolyldimethyl taurate / polysorbate 80 / sorbitan oleate) ; les copolymères d'acide 2-acrylamido 2-méthylpropane sulfonique et d'hydroxyethyl acrylate comme le SIMULGEL NS® et le SEPINOV EMT 10® commercialisés par la société SEPPIC ; les dérivés cellulosiques tels que l'hydroxyéthylcellulose ; les polysaccharides et notamment les gommes telles que la gomme de xanthane ; les dérivés siliconés hydrosolubles ou hydrodispersibles comme les silicones acryliques, les silicones polyéthers et les silicones cationiques et leurs mélanges.

Les tensioactifs sont de préférence choisi parmi les tensioactifs anioniques, cationiques, non-ioniques, zwitterioniques et amphotères. De préférence, ils sont choisis parmi :

a) les tensioactifs non ioniques, en particulier de HLB supérieure ou égale à 8 à 25°C, utilisés seuls ou en mélange. On peut citer notamment :

les esters et éthers d'oses tels que le mélange de cétylstéaryl glucoside et d'alcools cétylique et stéarylique comme le Montanov 68 de Seppic;

les éthers oxyéthylénés et/ou oxypropylénés (pouvant comporter de 1 à 150 groupes oxyéthylénés et/ou oxypropylénés) de glycérol ;

les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool cétéarylique à 30 groupes oxyéthylénés (nom CTFA « Ceteareth-30 »), l'éther oxyéthyléné de l'alcool stéarylique à 20 groupes oxyéthylénés (nom CTFA « Stéareth-20 »), l'éther oxyéthyléné du mélange d'alcools gras en C12-C15 comportant 7 groupes oxyéthylénés (nom CTFA « C12-15 Pareth-7 ») notamment commercialisé sous la dénomination NEODOL 25-7® par SHELL CHEMICALS ;

les esters d'acides gras polyoxyalkylénés (en particulier polyoxyéthylénés et/ou polyoxypropylénés) éventuellement en association avec un ester d'acide gras et de glycérol comme le mélange PEG-100 Stearate/Glyceryl Stearate commercialisé par exemple par la société ICI sous la dénomination Arlacel 165 ;

les esters d'acide gras (notamment d'acide en C8-C24, et de préférence en C16-C22) et des éthers de glycérol oxyéthylénés et/ou oxypropylénés (pouvant comporter de 1 à 150 groupes oxyéthylénés et/ou oxypropylénés), comme le monostéarate de PEG-200 glycéryle notamment vendu sous la dénomination Simulsol 220 TM® par la société SEPPIC ; le stéarate de PEG-50 et le monostéarate de PEG-40 notamment, commercialisé sous le nom MYRJ 52P® par la société ICI UNIQUEMA ; le stéarate de glycéryle polyéthoxylé à 30 groupes d'oxyde d'éthylène comme le produit TAGAT S® vendu par la société Evonik GOLDSCHMIDT, l'oléate de glycéryle polyéthoxylé à 30 groupes d'oxyde d'éthylène comme le produit TAGAT O® vendu par la société Evonik GOLDSCHMIDT, le cocoate de glycéryle polyéthoxylé à 30 groupes d'oxyde d'éthylène comme le produit VARIONIC LI 13® vendu par la société SHEREX, l'isostéarate de glycéryle polyéthoxylé à 30 groupes d'oxyde d'éthylène comme le produit TAGAT L® vendu par la société Evonik GOLDSCHMIDT et le laurate de glycéryle polyéthoxylé à 30 groupes d'oxyde d'éthylène comme le produit TAGAT I® de la société Evonik GOLDSCHMIDT,

les esters d'acide gras (notamment d'acide en C8-C24, et de préférence en C16-C22) et des éthers de sorbitol oxyéthylénés et/ou oxypropylénés (pouvant comporter de 1 à 150 groupes oxyéthylénés et/ou oxypropylénés), comme le polysorbate 20 notamment vendu sous la dénomination Tween 20® par la société CRODA, le polysorbate 60 notamment vendu sous la dénomination Tween 60® par la société CRODA,

la diméthicone copolyol, telle que celle vendue sous la dénomination Q2-5220® par la société DOW CORNING,

la diméthicone copolyol benzoate (FINSOLV SLB 101® et 201® de la société FINTEX),

les copolymères d'oxyde de propylène et d'oxyde d'éthylène, également appelés polycondensats OE/OP,

et leurs mélanges.

b) les tensioactifs non ioniques de HLB inférieure à 8 à 25°C, notamment :

les esters et éthers d'oses tels que le stéarate de sucrose, le cocoate de sucrose, le stéarate de sorbitan et leurs mélanges comme l'Arlatone 2121®commercialisé par la société ICI ;

les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool stéarylique à 2 groupes oxyéthylénés (nom CTFA « Stéareth-2 ») ;

les esters d'acides gras (notamment d'acide en C8-C24, et de préférence en C16C22) et de polyol, notamment de glycérol ou de sorbitol, tels que le stéarate de glycéryle, tel que le produit vendu sous la dénomination TEGIN M® par la société Evonik GOLDSCHMIDT, le laurate de glycéryle tel que le produit vendu sous la dénomination IMWITOR 312® par la société HULS, le stéarate de polyglycéryl-2, le tristéarate de sorbitan, le ricinoléate de glycéryle ;

les lécithines, telles que les lécithines de soja (comme Emulmetik 100 J de Cargill, ou Biophilic H de Lucas Meyer) ;

le mélange de cyclométhicone/diméthicone copolyol vendu sous la dénomination Q2-3225C® par la société DOW CORNING.

c) les tensioactifs anioniques tels que :

les sels d'acides gras polyoxyéthylénés notamment ceux dérivant des amines ou les sels alcalins, et leurs mélanges ;

les esters phosphoriques et leurs sels tels que le « DEA oleth-10 phosphate » (Crodafos N 10N de la société CRODA) ou le phosphate de monocétyle monopotassique ou cétyl phosphate de potassium (Amphisol K de Givaudan) ;

les sulfosuccinates tels que le «Disodium PEG-5 citrate lauryl sulfosuccinate» et le « Disodium ricinoleamido

MEA sulfosuccinate » ;
les alkyléthersulfates tels que le lauryl éther sulfate de sodium ;
les iséthionates ;
les acylglutamates tels que le « Disodium hydrogenated tallow glutamate » (AMISOFT HS-21 R® commercialisé par la société AJINOMOTO) et le sodium stearoyl glutamate (AMISOFT HS-11 PF® commercialisé par la société AJINOMOTO) et leurs mélanges ;
les dérivés de soja comme le potassium soyate ;
les citrates, comme le Glyceryl stearate citrate (Axol C 62 Pellets de Degussa) ;
les dérivés de proline, comme le Sodium palmitoyl proline (Sepicalm VG de Seppic), ou le Mélange de Sodium palmitoyle sarcosinate, Magnesium palmitoyle glutamate, acide palmitique et Palmitoyle proline (Sepifeel One de Seppic) ;
les lactylates, comme le Sodium stearoyl lactylate (Akoline SL de Karlshamns AB) ;
les sarcosinates, comme le sodium palmitoyle sarcosinate (Nikkol sarcosinate PN) ou le mélange de Stéaroyle sarcosine et Myristoyle sarcosine 75/25 (Crodasin SM de Croda) ;
les sulfonates, comme le Sodium C14-17 alkyl sec sulfonate (Hostapur SAS 60 de Clariant) ;
les glycinates, comme le sodium cocoyle glycinate (Amilite GCS-12 d'Ajinomoto).

Parmi les agents acidifiants, on peut citer les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques. Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium.
Parmi les actifs pour le soin des matières kératiniques telles que la peau, les lèvres, le cuir chevelu, les cheveux, les cils ou les ongles, on peut citer par exemple :

- les vitamines et leurs dérivés ou précurseurs, seuls ou en mélanges,
- les agents anti-oxydants ;
- les agents anti-radicalaires ;
- les agents anti-polluants,
- les agents autobronzants,
- les agents anti-glycation ;
- les agents apaisants,
- les agents déodorants,
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants,
- les agents rafraîchissants,
- les agents tenseurs,
- les agents matifiants,
- les agents hydratants,
- les agents anti-inflammatoires ;
- les agents anti-microbiens,
- les agents amincissants,
- les agents agissant sur le métabolisme énergétique des cellules,
- les agents répulsifs contre les insectes,
- les antagonistes de substances P ou de CRGP
- les agents anti-chute des cheveux
- les agents anti-rides,
- les agents anti-vieillissement.

L'homme du métier choisira le ou lesdits actifs en fonction de l'effet recherché sur la peau, les cheveux, les cils, les sourcils ou les ongles.
Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.
Les compositions selon l'invention peuvent être préparées selon les techniques bien connues de l'homme de

l'art. Elles peuvent se présenter en particulier sous forme de gel ou d'émulsion, simple ou complexe (H/E, E/H, H/E/H ou E/H/E) telle qu'une crème, un lait ou d'un gel crème. Elles peuvent éventuellement être conditionnées en aérosol et se présenter sous forme de mousse ou de spray.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517.

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Un autre objet de la présente invention est constitué par un procédé cosmétique non-thérapeutique de soin et/ou de maquillage d'une matière kératinique consistant à appliquer sur la surface de ladite matière kératinique au moins une composition selon l'invention telle que définie ci-dessus.

Les exemples qui suivent servent à illustrer l'invention sans toutefois présenter un caractère limitatif. Dans ces exemples, les quantités des ingrédients compositions sont données en % pondéral par rapport au poids total de la composition.

**EXEMPLES**

**Exemple 1 : préparation de formules et évaluation de leur photo-instabilité**

Les formules suivantes 1 à 4 sont préparées selon le protocole suivant :
Les phases aqueuses et huileuses sont préparées par mélange des matières premières sous agitation mécanique à 80°C ; les solutions obtenues sont macroscopiquement homogènes.
L'émulsion est préparée par introduction lente de la phase huileuse dans la phase aqueuse sous agitation à l'aide d'un homogénéisateur de type Moritz sous une vitesse d'agitation de 4000 RPM pendant 15 minutes.
L'émulsion obtenue est refroidie à la température ambiante, puis les phases restantes y sont ajoutées sous agitation lente.
L'émulsion se caractérise par des gouttes de taille comprise entre 1 $\mu$m et 10 $\mu$m.
Les particules de TiO2 de l'exemple 4 hors invention sont introduites dans la phase grasse, alors que les particules à base de perlite selon l'invention et présentes dans les formules 1 à 3 selon l'invention sont introduites dans l'émulsion à température ambiante, donc après formation de m'émulsion.

La photo-instabilité des formules 1 à 4 préparées est évaluée comme suit :
L'effet photocatalytique du TiO2 en présence de la vitamine F dans le milieu est détecté par une augmentation de la formation du pentane. En fait, la vitamine F s'oxyde sous irradiation UV pour donner du pentane.
Des mélanges contenant de la vitamine F et des particules à base de TiO2 ont été préparés ; le témoin est le mélange avec seulement la vitamine F, sans TiO2.
L'échantillon est exposé aux UV à la dose de 60 J UVA/cm2. On dose la quantité de pentane dans le flacon avec et sans irradiation. La photo-instabilité est représentée par le ratio suivant :

Photo-Instabilité = (%Pentane en présence de la particule/%pentane sans la particule)*100

La particule est considérée très photo-instable quand le pourcentage est proche de 100%. La méthode de préparation des formules permettant de mesurer la photostabilité des particules est la suivante.
La phase aqueuse est chauffée à 65°C, l'émulsion est préparée par introduction lente de la phase huileuse dans la phase aqueuse sous agitation à l'aide d'un homogénéisateur de type Moritz sous une vitesse d'agitation de 4000 RPM pendant 15 minutes. L'émulsion obtenue est refroidie sous agitation à 40°C. L'émulsion obtenue est refroidie à la température ambiante, puis les phases restantes y sont ajoutées sous agitation lente La vitamine F et les particules sont introduite une par une.

La composition des formules 1 à 4 et les résultats de photo-instabilité sont présentés ci-dessous :
La composition 4 comprend 2% en matière active de TiO2.

| | | Exemple 1 Selon Invention | Exemple 2 Selon Invention | Exemple 3 Selon Invention | Exemple 4 Hors Invention |
|---|---|---|---|---|---|
| | INCI UE | | | | |
| | TRIETHANOLAMINE | 0,55 | 0,55 | 0,55 | 0,55 |
| | CI 77891 (TiO2) | | | | 2 |
| | Particule selon l'exemple de préparation A enrobée de 3% de silice (TITANIUM DIOXIDE PERLITE and 3% SILICA) | 10,38 | | | |
| | Particule selon l'exemple de préparation A enrobée de 4% de silice (TITANIUM DIOXIDE PERLITE and 4% SILICA) | | 10,375 | | |
| | Particule selon l'exemple de préparation A enrobée de 5% de silice (TITANIUM DIOXIDE PERLITE and 5% SILICA) | | | 10,38 | |
| | DIISOPROPYL SEBACATE | 4 | 4 | 4 | 4 |
| | XANTHAN GUM | 0,1 | 0,1 | 0,1 | 0,1 |
| | ACRYLATES/C10-30 ALKYL ACRYLATE CROSSPOLYMER **(PEMULEN TR-1 POLYMER)** | 0,25 | 0,25 | 0,25 | 0,25 |
| | ALCOHOL DENAT. | 5 | 5 | 5 | 5 |
| | Eau | Qsp100 | Qsp100 | Qsp100 | Qsp100 |
| | C12-15 ALKYL BENZOATE | 13 | 13 | 13 | 13 |
| | GLYCERIN | 7 | 7 | 7 | 7 |
| | STEARIC ACID | 1 | 1 | 1 | 1 |
| | GLYCERYLSTEARATE (and) PEG-100 STEARATE **(ARLACEL 165-FP-PA-(SG))** | 2 | 2 | 2 | 2 |
| | POTASSIUM CETYL PHOSPHATE **(AMPHISOL K)** | 1 | 1 | 1 | 1 |
| | LINOLEIC ACID (and) OLEIC ACID (and) LINOLENIC ACID **(VITAMINE F ACIDE)** | 6 | 6 | 6 | 6 |
| **Photo-Instabilité 60J** | | **72%** | **35%** | **14%** | **100%** |

Les résultats obtenus montrent donc que le TiO2 contenu dans une matrice de perlite et enrobé de silice selon l'invention est plus photostable, et que cette photostabilité est croissante en fonction de taux d'enrobage de silice.

**Exemple 2 : préparation de formules et évaluation de leur stabilité et du blanchiment à l'application**

Les formules 5 à 8 suivantes ont été préparées comme décrit à l'exemple 1.

La stabilité des formules 5 à 8 est évaluée comme suit :

La stabilité des compositions de l'invention est évaluée par observations microscopiques de leur aspect, spécifiquement l'état de dispersion des particules. Une composition est jugée stable lorsque que leur aspect microscopique et leur viscosité sont stables à T0 et pendant 1 mois à température ambiante (25°C).

Le blanchiment des formules 5 à 8 est évalué comme suit :

L'évaluation du blanchiment des formules est faite selon la méthode suivante :
2mg de la formule sont déposés sur une surface de tissu noir avec une rugosité proche de la peau. Le produit est étalé en appliquant un mouvement circulaire de 10 tours. Le produit est séché pendant 5min et le blanchiment est évalué, avec une notation de +++ pour le plus blanchissant et + pour le moins blanchissant.

La composition des formules 5 à 8 et les résultats de stabilité et blanchiment sont présentés ci-dessous :

| INCI UE | Exemple 5 Hors Invention | Exemple 6 Selon Invention | Exemple 7 Selon Invention | Exemple 8 Selon Invention |
|---|---|---|---|---|
| DISODIUM EDTA | 0,1 | 0,2 | 0,2 | 0,2 |
| TOCOPHEROL | 0,1 | 0,1 | 0,1 | 0,1 |
| TRIETHANOLAMINE | 0,71 | 0,71 | 0,71 | 0,71 |
| Conservateurs | Qs | Qs | Qs | Qs |
| ISOHEXADECANE | 4,5 | 4,5 | 4,5 | 4,5 |
| BUTYL METHOXYDIBENZOYLMET HANE **(PARSOL 1789)** | 3 | 3 | 3 | 3 |
| TITANIUM DIOXIDE (and) ALUMINUM HYDROXIDE (and) STEARIC ACID **(MICRO TITANIUM DIOXID E MT-100 TV)** | 3 | | | |
| ETHYLHEXYL TRIAZONE **(UVINUL T 150)** | 0,5 | 0,5 | 0,5 | 0,5 |
| TEREPHTHALYLIDENE DICAMPHOR SULFONIC ACID **(MEXORYL SX)** | 0,9 | 0,9 | 0,9 | 0,9 |
| OCTOCRYLENE **(SUNOBEL OCT)** | 7 | 7 | 7 | 7 |
| Particule selon l'exemple de préparation A enrobée de 3% de silice (TITANIUM DIOXIDE PERLITE and 3% SILICA) | | 6,23 | | |
| Particule selon l'exemple de préparation A enrobée de 4% de silice (TITANIUM DIOXIDE PERLITE and 4% SILICA) | | | 6,23 | |
| Particule selon l'exemple de préparation A enrobée de 5% de silice (TITANIUM DIOXIDE PERLITE and 5% SILICA) | | | | 6,23 |
| XANTHAN GUM | 0,1 | 0,1 | 0,1 | 0,1 |
| ACRYLATES/C10-30 ALKYL ACRYLATE CROSSPOLYMER **(PEMULEN TR-1 POLYMER)** | 0,25 | 0,25 | 0,25 | 0,25 |
| DIMETHICONE **(BELSIL DM 350)** | 0,5 | 0,5 | 0,5 | 0,5 |
| ALCOHOL DENAT. | 2 | 2 | 2 | 2 |
| Eau | Qsp100 | Qsp100 | Qsp100 | Qsp100 |
| C12-15 ALKYL BENZOATE **(FINSOLV TN)** | 7,5 | 7,5 | 7,5 | 7,5 |
| GLYCERIN | 6 | 6 | 6 | 6 |
| PROPYLENE GLYCOL | 6 | 6 | 6 | 6 |
| STEARIC ACID | 1 | 1 | 1 | 1 |
| GLYCERYL STEARATE (and) PEG-100 STEARATE **(SIMULSOL 165)** | 1 | 1 | 1 | 1 |

(suite)

| INCI UE | Exemple 5 Hors Invention | Exemple 6 Selon Invention | Exemple 7 Selon Invention | Exemple 8 Selon Invention |
|---|---|---|---|---|
| POTASSIUM CETYL PHOSPHATE | 1 | 1 | 1 | 1 |
| **(AMPHISOL K)** | | | | |
| **Blanchiment** | +++ | + | + | + |
| **Stabilité 2 mois 40°C et 45°C** | Stable | Stable | stable | stable |

Les résultats montrent les mesures du blanchiment *in vitro* de formules comprenant 2.5% en poids de TiO2 en matière active.
En revanche, le blanchiment est beaucoup moins important avec les particules de l'invention (formules 6 à 8).
En outre, les compositions selon l'invention présentent un fort SPF et une forte pigmentation immédiate persistante (ou « persistent pigmentation darkening », ou PPD, ciblée sur les UVA).

## Revendications

1. Composition comprenant, notamment dans un milieu cosmétiquement acceptable :

    a) au moins une phase aqueuse ; et
    b) des particules composites de taille moyenne élémentaire en nombre supérieure à 0,1 μm comprenant :

        i) une matrice en perlite,
        ii) au moins un filtre UV inorganique, et
        iii) un enrobage de silice, ladite silice représentant au moins 1% en poids par rapport au poids total de la particule composite.

2. Composition selon la revendication 1, **caractérisée en ce que** les particules composites présentent une taille moyenne élémentaire en nombre variant entre 0,1 et 30 μm, de préférence entre 1 et 28 μm, mieux entre 3 et 25 μm.

3. Composition selon la revendication 1 ou 2, **caractérisée en ce que** les particules composites sont présentes dans des concentrations allant de 1 à 70%, de préférence 1,5 à 50%, de manière préférée de 2 à 40% en poids par rapport au poids total de la composition.

4. Composition selon l'une des revendications précédentes, **caractérisée en ce que** le filtre UV inorganique est choisi parmi les oxydes métalliques, de préférence les oxydes de titane, de zinc, de fer ou leurs mélanges, et plus particulièrement parmi le dioxyde de titane, l'oxyde de zinc et leurs mélanges.

5. Composition selon l'une des revendications précédentes, **caractérisée en ce que** le filtre UV inorganique est présent dans les particules composites en une teneur allant de 1% à 70% en poids, mieux de 1,2% à 65% en poids, et encore mieux de 1,5% à 60% en poids par rapport au poids total d'une particule composite.

6. Composition selon l'une des revendications précédentes, **caractérisée en ce que** la perlite est présente dans les particules composites en une teneur allant de 10 à 99% en poids, plus préférentiellement de 20 à 98% en poids et encore plus préférentiellement 30 à 97% en poids par rapport au poids total d'une particule composite.

7. Composition selon l'une des revendications précédentes, **caractérisée en ce que** l'enrobage de silice représente au moins 2% en poids par rapport au poids total d'une particule composite.

8. Composition selon l'une des revendications 1 à 6, **caractérisée en ce que** l'enrobage de silice représente de 1 à 10% en poids, plus préférentiellement de 2 à 8% en poids et encore plus préférentiellement de 2,5 à 6% en poids par rapport au poids total d'une particule composite.

9. Composition selon l'une des revendications précédentes, **caractérisée en ce que** la phase aqueuse représente

de 15 à 95% en poids, préférentiellement de 30 à 80% en poids, encore mieux de 40 à 70% en poids par rapport au poids total de la composition.

10. Composition selon l'une des revendications précédentes, **caractérisée en ce qu'**elle comprend au moins une phase huileuse, de préférence au moins une huile volatile ou non volatile.

11. Composition selon l'une des revendications précédentes, **caractérisée en ce qu'**elle comprend au moins un filtre UV organique, de préférence en une teneur allant de 0,01 à 30% en poids et de préférence de 0,1 à 20% en poids par rapport au poids total de la composition.

12. Procédé cosmétique non thérapeutique de soin et/ou de maquillage des matières kératiniques humaines, notamment la peau du corps ou du visage ou des cheveux, comprenant au moins l'application sur lesdites matières kératiniques d'une composition selon l'une des revendications précédentes.

## Patentansprüche

1. Zusammensetzung, umfassend, insbesondere in einem kosmetisch annehmbaren Umfeld:

   a) mindestens eine wässrige Phase; und
   b) Verbundpartikel einer zahlenmittleren Elementargröße von mehr als 0,1 μm, umfassend:

      i) eine Perlitmatrix,
      ii) mindestens einen anorganischen UV-Filter, und
      iii) eine Beschichtung aus Siliciumdioxid, wobei das Siliciumdioxid mindestens 1 Gewichtsprozent, bezogen auf das Gesamtgewicht des Verbundpartikels, darstellt.

2. Zusammensetzung nach Anspruch 1, **dadurch gekennzeichnet, dass** die Verbundpartikel eine zahlenmittlere Elementargröße aufweisen, die zwischen 0,1 und 30 μm, vorzugsweise zwischen 1 und 28 μm, besser zwischen 3 und 25 μm variiert.

3. Zusammensetzung nach Anspruch 1 oder 2, **dadurch gekennzeichnet, dass** die Verbundpartikel in Konzentrationen in einem Bereich von 1 bis 70 %, vorzugsweise 1,5 bis 50 %, bevorzugt 2 bis 40 Gewichtsprozent, bezogen auf das Gesamtgewicht der Zusammensetzung, vorhanden sind.

4. Zusammensetzung nach einem der vorherigen Ansprüche, **dadurch gekennzeichnet, dass** der anorganische UV-Filter ausgewählt ist aus den Metalloxiden, vorzugsweise dem Titan-, Zink-, Eisenoxid oder deren Gemischen, und insbesondere aus Titandioxid, Zinkoxid und deren Gemischen.

5. Zusammensetzung nach einem der vorherigen Ansprüche, **dadurch gekennzeichnet, dass** der anorganische UV-Filter in den Verbundpartikeln in einem Gehalt von 1 bis 70 Gewichtsprozent, besser von 1,2 bis 65 Gewichtsprozent und noch besser von 1,5 bis 60 Gewichtsprozent, bezogen auf das Gesamtgewicht eines Verbundpartikels, vorhanden ist.

6. Zusammensetzung nach einem der vorherigen Ansprüche, **dadurch gekennzeichnet, dass** das Perlit in den Verbundpartikeln in einem Gehalt von 10 bis 99 Gewichtsprozent, bevorzugter von 20 bis 98 Gewichtsprozent und noch bevorzugter von 30 bis 97 Gewichtsprozent, bezogen auf das Gesamtgewicht eines Verbundpartikels, vorhanden ist.

7. Zusammensetzung nach einem der vorherigen Ansprüche, **dadurch gekennzeichnet, dass** die Siliciumdioxidbeschichtung mindestens 2 Gewichtsprozent, bezogen auf das Gesamtgewicht eines Verbundpartikels, darstellt.

8. Zusammensetzung nach einem der Ansprüche 1 bis 6, **dadurch gekennzeichnet, dass** die Siliciumdioxidbeschichtung 1 bis 10 Gewichtsprozent, bevorzugter 2 bis 8 Gewichtsprozent und noch bevorzugter 2,5 bis 6 Gewichtsprozent, bezogen auf das Gesamtgewicht eines Verbundpartikels, darstellt.

9. Zusammensetzung nach einem der vorherigen Ansprüche, **dadurch gekennzeichnet, dass** die wässrige Phase 15 bis 95 Gewichtsprozent, vorzugsweise 30 bis 80 Gewichtsprozent, noch besser 40 bis 70 Gewichtsprozent,

bezogen auf das Gesamtgewicht der Zusammensetzung, darstellt.

**10.** Zusammensetzung nach einem der vorherigen Ansprüche, **dadurch gekennzeichnet, dass** sie mindestens eine Ölphase, vorzugsweise mindestens ein flüchtiges oder nicht flüchtiges Öl, umfasst.

**11.** Zusammensetzung nach einem der vorherigen Ansprüche, **dadurch gekennzeichnet, dass** sie mindestens einen organischen UV-Filter umfasst, vorzugsweise in einem Gehalt von 0,01 bis 30 Gewichtsprozent und vorzugsweise von 0,1 bis 20 Gewichtsprozent, bezogen auf das Gesamtgewicht der Zusammensetzung.

**12.** Nicht-therapeutisches kosmetisches Verfahren zum Pflegen und/oder Schminken menschlicher Keratinmaterialien, insbesondere der Körper- oder Gesichtshaut oder der Haare, mindestens umfassend das Auftragen einer Zusammensetzung nach einem der vorherigen Ansprüche auf die Keratinmaterialien.

## Claims

**1.** Composition comprising, particularly in a cosmetically acceptable medium:

a) at least one aqueous phase; and
b) composite particles with a number-average elementary size greater than 0.1 μm comprising:

i) a perlite matrix,
ii) at least one inorganic UV filter, and
iii) a silica coating, said silica representing at least 1% by weight relative to the total weight of the composite particle.

**2.** Composition according to claim 1, **characterized in that** the composite particles have a number-average elementary size varying between 0.1 and 30 μm, preferably between 1 and 28 μm, more preferentially between 3 and 25 μm.

**3.** Composition according to claim 1 or 2, **characterized in that** the composite particles are present in concentrations ranging from 1 to 70%, preferably from 1.5 to 50%, preferentially from 2 to 40% by weight relative to the total weight of the composition.

**4.** Composition according to one of the preceding claims, **characterized in that** the inorganic UV filter is chosen among metal oxides, preferably titanium, zinc, iron oxides or mixtures thereof, and more particularly among titanium dioxide, zinc oxide and mixtures thereof.

**5.** Composition according to one of the preceding claims, **characterized in that** the inorganic UV filter is present in the composite particles in a content ranging from 1% to 70% by weight, preferably from 1.2% to 65% by weight, and more preferably from 1.5% to 60% by weight relative to the total weight of a composite particle.

**6.** Composition according to one of the preceding claims, **characterized in that** perlite is present in the composite particles in a content ranging from 10 to 99% by weight, more preferentially from 20 to 98% by weight and more preferentially from 30 to 97% by weight relative to the total weight of a composite particle.

**7.** Composition according to one of the preceding claims, **characterized in that** the silica coating represents at least 2% by weight relative to the total weight of a composite particle.

**8.** Composition according to one of claims 1 to 6, **characterized in that** the silica coating represents from 1 to 10% by weight, more preferentially from 2 to 8% by weight and even more preferentially from 2.5 to 6% by weight relative to the total weight of a composite particle.

**9.** Composition according to one of the preceding claims, **characterized in that** the aqueous phase represents from 15 to 95% by weight, preferentially from 30 to 80% by weight, more preferably from 40 to 70% by weight relative to the total weight of the composition.

**10.** Composition according to one of the preceding claims, **characterized in that** it comprises at least one oily phase, preferably at least one volatile or non-volatile oil.

11. Composition according to one of the preceding claims, **characterized in that** it comprises at least one organic UV filter, preferably in a content ranging from 0.01 to 30% by weight and preferably from 0.1 to 20% by weight relative to the total weight of the composition.

12. Non-therapeutic cosmetic process for the treatment and/or makeup of human keratin materials, particularly the skin of the body or face or hair, comprising at least the application on said keratin materials of a composition according to one of the preceding claims.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5562990 A
- US 5002698 A
- FR 2882371 B1
- WO 2006083326 A
- KR 1020000069638
- JP 2008115161 B
- WO 2007068371 A
- WO 2008155059 A
- EP 669323 A
- US 2463264 A
- US 5166355 A
- GB 2303549 A
- DE 19726184
- EP 893119 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- WO 2007071584 A
- WO 2009063392 A
- WO 2004085412 A
- WO 06035000 A
- WO 06034982 A
- WO 06034991 A
- WO 06035007 A
- WO 2006034992 A
- WO 2006034985 A
- EP 0841341 A
- US 4077441 A
- US 4850517 A


### Littérature non-brevet citée dans la description


- **VAN DE HULST, H.C.** Light Scattering by Small Particles. Wiley, 1957
- *CHEMICAL ABSTRACTS,* 919803-06-8
- Symetrical Triazine Derivatives. INC WEST HENRIETTA, 20 Septembre 2004